**Europäisches Patentamt**

(19) **European Patent Office**

**Office européen des brevets**

(11) Publication number : **0 536 308 B1**

(12) # EUROPEAN PATENT SPECIFICATION

(45) Date of publication of patent specification :
**02.02.94 Bulletin 94/05**

(51) Int. Cl.[5] : **D01D 5/253,** A61L 15/00, A61F 13/15

(21) Application number : **91913295.1**

(22) Date of filing : **24.06.91**

(86) International application number :
**PCT/US91/04446**

(87) International publication number :
**WO 92/00407 09.01.92 Gazette 92/02**

(54) **ABSORBANT FIBERS CAPABLE OF SPONTANEOUSLY TRANSPORTING FLUIDS.**

(30) Priority : **28.06.90 US 545221**

(43) Date of publication of application :
**14.04.93 Bulletin 93/15**

(45) Publication of the grant of the patent :
**02.02.94 Bulletin 94/05**

(84) Designated Contracting States :
**AT BE CH DE DK ES FR GB GR IT LI LU NL SE**

(56) References cited :
**EP-A- 0 188 091**
**EP-A- 0 391 814**

(73) Proprietor : **EASTMAN KODAK COMPANY**
**343 State Street**
**Rochester, New York 14650-2201 (US)**

(72) Inventor : **PHILLIPS, Bobby, Mal**
**Route 2, Box 253G**
**Jonesborough, TN 37659 (US)**
Inventor : **DALTON, James, Samuel, Nelson**
**2121 Bonaire**
**Kingsport, TN 37660 (US)**

(74) Representative : **Buff, Michel et al**
**Kodak-Pathé Département des Brevets et**
**Licences CRT Centre de Recherches et de**
**Technologie Zone Industrielle**
**F-71102 Chalon sur Saône Cédex (FR)**

## Description

This application is a continuation-in-part of application Serial No. 333,651, filed April 4, 1989.

Field of the Invention

This invention concerns absorbant fibers that are capable of spontaneously transporting water on their surfaces and useful structures made from such fibers.

Background of the Invention

Presently available absorbant articles such as diapers, sanitary napkins, incontinence briefs, and the like are generally very good at absorbing aqueous fluids such as urine and blood. However, during typical use such articles become saturated at the impingement zone while other zones removed from the impingement zone will remain dry. As a result, a substantial portion of the total absorbant capabilities of such articles remains unused. Thus, it would be highly desirable to have a means for transporting the aqueous fluids from the impingement zone to other areas of the absorbant article to more fully utilize the article's total absorbant capability. In copending application Serial No. 333,651, incorporated herein by reference in its entirety, such a means of fluid transport by use of certain fibers is disclosed.

The ability to transport liquids (alternately referred to herein as "wickability") and to hold liquids are two important features of absorbant cores of sanitary consumer disposables such as diapers, adult incontinent products, and feminine hygiene products. Absorbant cores are designed to wick fluids as far as possible to prevent leakage and optimize the use of absorbant material. In a conventional diaper, fluid is wicked by capillary action through the porous fluff pulp core. Liquid holding capacity is largely within the pores of the fluff pulp but is also enhanced by the addition of superabsorbant polymers to the absorbant core. These superabsorbant polymers are especially beneficial for holding liquids under pressure compared to pulp alone. Absorbant cores of diapers and adult incontinent products do not sufficiently wick fluids from the crotch area to entirely prevent leaking. Typically 3-7% of diapers and 33-40% of adult incontinent products leak. Leaking is the number one customer complaint about these products. Solving the leaking problem is high priority among the manufacturers of these products.

In the prior art thermally bonded webs composed of polyester, polypropylene, or polyethylene hydrophobic fibers are formed. These webs are subsequently coated with acrylic acid partially neutralized by alkali metallic salts and crosslinked simultaneously with polymerization to form webs coated in situ with superabsorbant polymer (European Patent Application 0 223 908). The webs have increased absorption of fluid when used in a sanitary product such as a diaper, but the individual fibers of the web do not possess the ability to wick fluid from the crotch area (which is most prone to leaking) to lesser utilized areas of the absorbant core.

Japanese Patent Laid-Open No. 204,975/1984 describes the coating of cellulose fiber based material with a water soluble monomer which is converted into a water-absorptive polymer. According to U.S. Patent 4,721,647 this type of material has poor absorption performance because the monomer is able to penetrate inside the fiber base material and fill the capillaries between filaments. The mode of wicking in this prior art is totally in the capillaries between the fibers. The diameter of the capillaries is reduced by the coating. As the coating swells in the wet state the capillaries are blocked off. EP-A-188 091 discloses highly absorbent non-woven webs formed from readily available nonwoven materials and having a polyelectrolyte super-absorbent polymeric sorbent coating the individual fibers of the nonwoven web. The polymeric sorbent coating absorbs liquid while minimizing occlusion of the interstices of the web.

We have discovered spontaneously transportable fibers coated with superabsorbing polymers which are capable of absorbing liquid as well as transporting liquid.

Summary of the Invention

The present invention is directed to a synthetic absorbant, coated, fiber which is capable of spontaneously transporting water on the surface thereof.

The fiber of the present invention has at least one continuous groove oriented axially along the fiber and said fiber satisfies the following equation

$$(1 - X \cos \theta_a) < 0,$$

wherein

$\theta_a$ is the advancing contact angle of water measured on a flat film made from the same material as the fiber and having the same surface treatment, if any,

X is a shape factor of the fiber cross-section that satisfies the following equation

$$X = \frac{P_w}{4r + (\pi - 2)D}$$

wherein

$P_w$ is the wetted perimeter of the fiber and r is the radius of the circumscribed circle circumscribing the fiber cross-section and D is the minor axis dimension across the fiber cross-section, wherein said fiber has coated thereon at least one superabsorbent polymer.

It is preferred that the fiber of the invention satisfies the formula:

$$\gamma_{LA} \cdot \frac{12\pi \cdot 10^{-4}}{\sqrt{\rho}} \cdot \sqrt{dpf} \cdot (1 - X \cos \theta_a) \leq -0.3,$$

wherein $\gamma_{LA}$ is the surface tension of water in air in dynes/cm, $\rho$ is the fiber density in grams/cc, and dpf is the denier of the single fiber.

It is preferred that X is greater than 1.2, more preferably greater than about 2.5, most preferably greater than about 4.

## Brief Description of the Drawings

Figure 1 - schematic representation of a three dimensional view of an absorbant fiber of the invention illustrating the swelling of superabsorbant material out of a fiber groove upon transport of a fluid.

Figure 2A - illustration of the behavior of a drop of an aqueous fluid which has just contacted a fiber that is spontaneously transportable at time = 0. The arrows labelled "LFA" indicate the location of the liquid-fiber-air interface.

Figure 2B - illustration of the behavior of a drop of an aqueous fluid on a fiber that is spontaneously transportable at time = $t_1$ ($t_1$ >0). The arrows labelled "LFA" indicate the location of the liquid-fiber-air interface.

Figure 2C - illustration of the behavior of a drop of an aqueous fluid on a fiber that is spontaneously transportable at time = $t_2$ ($t_2$ >$t_1$). The arrows labelled "LFA" indicate the location of the liquid-fiber-air interface.

Figure 3 - schematic representation of an orifice of a spinneret useful for producing a spontaneously transportable fiber.

Figure 4 - schematic representation of an orifice of a spinneret useful for producing a spontaneously transportable fiber.

Figure 5 - schematic representation of an orifice of a spinneret useful for producing a spontaneously transportable fiber.

Figure 6 - schematic representation of an orifice of a spinneret useful for producing a spontaneously transportable fiber.

Figure 6B - schematic representation of an orifice of a spinneret useful for producing a spontaneously transportable fiber.

Figure 7 - schematic representation of an orifice of a spinneret having 2 repeating units, joined end to end, of the orifice as shown in Figure 3.

Figure 8 - schematic representation of an orifice of a spinneret having 4 repeating units, joined end to end, of the orifice as shown in Figure 3.

Figure 9 - photomicrograph of a poly(ethylene terephthalate) fiber cross-section made using a spinneret having an orifice as illustrated in Figure 3 (specific dimensions of spinneret orifice described in Example 1).

Figure 10 - photomicrograph of a polypropylene fiber cross-section made using a spinneret having an orifice as illustrated in Figure 3 (specific dimensions of spinneret orifice described in Example 2).

Figure 11 - photomicrograph of a nylon 66 fiber cross-section made using a spinneret having an orifice as illustrated in Figure 3 (specific dimensions of spinneret orifice described in Example 2).

Figure 12 - schematic representation of a poly(ethylene terephthalate) fiber cross-section made using a spinneret having an orifice as illustrated in Figure 4 (specific dimensions of spinneret orifice described in Example 8).

Figure 13 - photomicrograph of a poly(ethylene terephthalate) fiber cross-section made using a spinneret having an orifice as illustrated in Figure 5 (specific dimensions of spinneret orifice described in Example 9).

Figure 14 - photomicrograph of a poly(ethylene terephthalate) fiber cross-section made using a spinneret having an orifice as illustrated in Figure 7 (specific dimensions of spinneret orifice described in Example 10).

Figure 15 - photomicrograph of a poly(ethylene terephthalate) fiber cross-section made using a spinneret having an orifice as illustrated in Figure 8 (specific dimensions of spinneret orifice described in Example 11).

Figure 16 - schematic representation of a fiber cross-section made using a spinneret having an orifice as illustrated in Figure 3 (Example 1). Exemplified is a typical means of determining the shape factor X.

Figure 17 - photomicrograph of a poly(ethylene terephthalate) fiber cross-section made using a spinneret having an orifice as illustrated in Figure 6 (specific dimensions of spinneret orifice described in Example 12).

Figure 17B - schematic representation of a poly(ethylene terephthalate) fiber cross-section made using a spinneret having an orifice as illustrated in Figure 6B (specific dimensions of spinneret orifice described in Example 13).

Detailed Description of the Invention

As used herein, the term "base fibers" means the fibers disclosed in Serial No. 333,651 not having a superabsorbant polymer coating (but optionally having a different surface treatment, e.g., a coating of a hydrophilic lubricant), and the terms "coated fiber", "absorbant fiber", or "coated, absorbant fiber" mean a fiber of the present invention, i.e., a base fiber having coated thereon at least one superabsorbant polymer.

The three important variables fundamental to the liquid transport behavior are (a) surface tension of the liquid, (b) wettability or the contact angle of the solid with the liquid, and (c) the geometry of the solid surface. Typically, the wettability of a solid surface by a liquid can be characterized by the contact angle that the liquid surface (gas-liquid interface) makes with the solid surface (gas-solid surface). Typically, a drop of liquid placed on a solid surface makes a contact angle, $\theta$, with the solid surface. If this contact angle is less than 90°, then the solid is considered to be wet by the liquid. However, if the contact angle is greater than 90°, such as with water on Teflon (trademark) surface, the solid is not wet by the liquid. Thus, it is desired to have a minimum contact angle for enhanced wetting, but definitely, it must be less than 90°. However, the contact angle also depends on surface inhomogeneities (chemical and physical, such as roughness), contamination, chemical/physical treatment of the solid surface, as well as the nature of the liquid surface and its contamination. Surface free energy of the solid also influences the wetting behavior. The lower the surface energy of the solid, the more difficult it is to wet the solid by liquids having high surface tension. Thus, for example, Teflon, which has low surface energy does not wet with water. (Contact angle for Teflon-water system is 112°.) However, it is possible to treat the surface of Teflon with a monomolecular film of protein, which significantly enhances the wetting behavior. Thus, it is possible to modify the surface energy of fiber surfaces by appropriate lubricants/finishes to enhance liquid transport. The contact angle of polyethylene terephthalate (PET), nylon 66, and polypropylene with water is 80°, 71°, and 108°, respectively. Thus, nylon 66 is more wettable than PET. However, for polypropylene, the contact angle is >90°, and thus is nonwettable with water.

The second property of fundamental importance to the phenomena of liquid transport is surface tension of the liquid.

The third property of fundamental importance to the phenomena of liquid transport is the geometry of the solid surface. Although it is known that grooves enhance fluid transport in general, we have discovered particular geometries and arrangements of deep and narrow grooves on fibers and treatments thereof which allow for the spontaneous surface transport of aqueous fluids in single fibers. Thus we have discovered fibers with a combination of properties wherein an individual fiber is capable of spontaneously transporting water on its surface.

The particular geometry of the deep and narrow grooves is very important. For example, in grooves which have the feature that the width of the groove at any depth is equal to or less than the width of the groove at the mouth of the groove are preferred over those grooves which do not meet this criterion. If the preferred groove is not achieved "bridging" of the liquid across the restriction is possible and thereby the effective wetted perimeter (Pw) is reduced. Accordingly, it is preferred that Pw is substantially equal to the geometric perimeter.

The number of continuous grooves present in the fiber of the present invention is not critical as long as the required geometry is present (i.e., the fiber satisfies the equation (1-X cos $\theta_a$) <0). Typically there are at least 2 grooves present, and preferably less than 10.

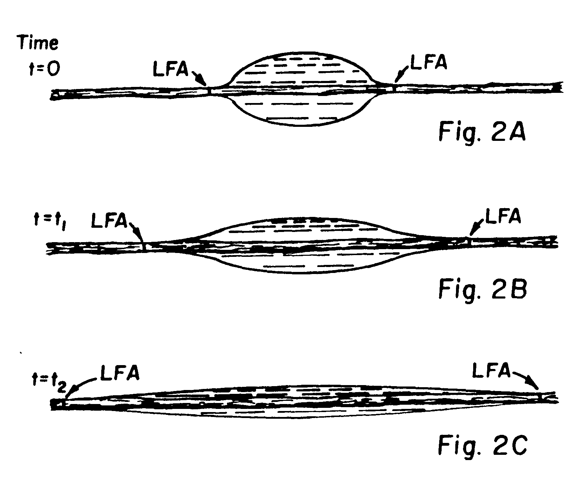

"Spontaneously transportable" and derivative terms thereof refer to the behavior of a fluid in general and in particular a drop of fluid, typically water, when it is brought into contact with a single fiber such that the drop spreads along the fiber. Such behavior is contrasted with the normal behavior of the drop which forms a static ellipsoidal shape with a unique contact angle at the intersection of the liquid and the solid fiber. It is obvious that the formation of the ellipsoidal drop takes a very short time but remains stationary thereafter. Figures 2A, 2B and 2C illustrate spontaneous fluid transport on a fiber surface. The key factor is the movement of the location of the air, liquid, solid interface with time. If such interface moves just after contact of the liquid with the fiber, then the fiber is spontaneously transportable; if such interface is stationary, the fiber is not spontaneously transportable. The spontaneously transportable phenomenon is easily visible to the naked eye for large filaments (>20 denier per filament (dpf) or >22.22 dtex) but a microscope may be necessary to view the fibers if they are less than 20 dpf (<22.22 dtex). Colored fluids are more easily seen but the spontaneously transportable phenomenon is not dependent on the color. It is possible to have sections of the circumference of the fiber on which the fluid moves faster than other sections. In such case the air, liquid, solid interface actually extends over a length of the fiber. Thus, such fibers are also spontaneously transportable in that the air, liquid, solid interface is moving as opposed to stationary.

Spontaneous transportability is basically a surface phenomenon; that is the movement of the fluid occurs on the surface of the fiber. However, it is possible and may in some cases be desirable to have the spontaneously transportable phenomenon occur in conjunction with absorption of the fluid into the fiber. The behavior visible to the naked eye will depend on the relative rate of absorption vs. spontaneous transportability. For example, if the relative rate of absorption is large such that most of the fluid is absorbed into the fiber, the liquid drop will disappear with very little movement of the air, liquid, solid interface along the fiber surface whereas if the rate of absorption is small compared to the rate of spontaneous transportability the observed behavior will be that of wicking or transport, as exemplified in Figures 2A through 2C. In Figure 2A, a drop of aqueous fluid is just placed on the fiber (time = 0). In Figure 2B, a time interval has elapsed (time = $t_1$) and the fluid starts to be spontaneously transported. In Figure 2C, a second time interval has passed (time = $t_2$) and the fluid has been spontaneously transported along the fiber surface further than at time = $t_1$.

A base fiber or a coated fiber of the present invention is capable of spontaneously transporting water on the surface thereof. Distilled water can be employed to test the spontaneous transportability phenomenon; however, it is often desirable to incorporate a minor amount of a colorant into the water to better visualize the spontaneous transport of the water, so long as the water with colorant behaves substantially the same as pure water under test conditions. We have found aqueous Syltint Poly Red (trademark) from Milliken Chemicals to be a useful solution to test the spontaneous transportability phenomenon. The Syltint Poly Red solution can be used undiluted or diluted significantly, e.g., up to about 50x with water.

In addition to being capable of transporting water, a base fiber or a coated fiber of the present invention is also capable of spontaneously transporting a multitude of other aqueous fluids. Aqueous fluids are those fluids comprising about 50% or more water by weight, preferred is about 75% or more water by weight, most preferred is about 90% or more water by weight. Preferred aqueous fluids are body fluids, especially human body fluids. Such preferred fluids include, but are not limited to, blood, urine, perspiration, and the like. Other preferred aqueous fluids include, for example, aqueous inks.

In addition to being able to transport aqueous fluids, a base fiber or coated fiber of the present invention is also capable of transporting an alcoholic fluid on its surface. Alcoholic fluids are those fluids comprising greater than about 50% by weight of an alcoholic compound of the formula

$$R\text{-}OH$$

wherein R is an aliphatic or aromatic group containing up to 12 carbon atoms. It is preferred that R is an alkyl group of 1 to 6 carbon atoms, more preferred is 1 to 4 carbon atoms. Examples of alcohols include methanol, ethanol, n-propanol and iso-propanol. Preferred alcoholic fluids comprise about 70% or more by weight of a suitable alcohol. Preferred alcoholic fluids include antimicrobial agents, such as disinfectants, and alcohol-based inks.

The superabsorbant coating of the coated fiber of the present invention acts as a "sink" and absorbs whatever fluid is being transported.

The absorbant fibers of the present invention are coated with at least one superabsorbant material. By the word "coated" and derivative terms thereof is meant that the superabsorbant material is in a continuous phase and completely surrounds the circumference of a fiber cross-section for at least a portion of the fiber length. Different embodiments of the coating include wherein the entire fiber is substantially coated and wherein the fiber is only intermittently coated. This intermittent coating provides segments which will wick fluid without absorbing it to areas which are coated with superabsorbant polymer and will absorb the fluid in a preferred area. Which specific embodiment is preferred will depend upon the particular desired application. A particular preferred embodiment is wherein the fiber of the present invention is substantially the length of an absorbant article (e.g., a diaper, an incontinent pad, or the like) and is coated on the ends of the fiber, but not in the center portion.

Also, in the coated fibers of the invention, the coating is in intimate contact with at least a portion of the fiber surface. Preferably, substantially the entire coating which is positioned adjacent to the fiber surface is in intimate contact with that portion of the fiber surface. That is, preferably all the groove surfaces are "filled" and no visible gaps appear between the coating and the fiber surface upon routine examination by microscopy at a magnification of about 20X.

Water soluble polymerizable monomers such as acrylic acid, methacrylic acid, and vinylsulfonic acid of which 20% or more of the carboxyl groups have been neutralized into an alkali metal salt can be used to form the superabsorbant coating on the base fibers. Preferred superabsorbant polymers are those formed which have a crosslinked structure. Water soluble crosslinking agents having two or more functional groups capable of reacting with a functional group of the aforementioned acids can be used. They are well known in the art. N,N'-methylene bisacrylamide, ethylene glycol bisacrylate, and polyglycidyl ethers are typical examples. The polymerization is carried out <u>in situ</u>, i.e., in the presence of the base fibers. The polymerization can be accomplished through thermal, light, accelerated electron beams, radiation, ultraviolet rays. It is necessary to add a water soluble radical polymerization initiator, in thermal polymerization, or a water-soluble initiator capable of generating radicals with the aid of light or ultraviolet rays in photopolymerization or ultra violet polymerization to the aqueous monomer solution. Initiators are well known in the art (see U.S. Patent 4,721,647). The degree of crosslinking can be varied to control the amount and rate of absorption to the extent that the superabsorbant polymer remains water insoluble. The amount of superabsorbant polymer coating can be varied. It is preferred that the amount be limited so that individual filaments are not bonded together or that the swollen gel is prevented from leaving the grooves of the filaments.

Generally, the methodology taught in U.S. Patent 4,721,647 (incorporated herein by reference in its entirety) and European Patent Application 0 188 091 can be used to prepare the coated absorbant fibers of the present invention except that one would substitute the spontaneously transportable fibers of Serial No. 333,651, (i.e., the base fibers) for the fiber used in the prior art methods.

A prior art example (European Patent Application 0 188 091) discloses non-woven webs having a thin superabsorbant polymeric coating on the individual fibers of the web. The fibers of this web are round cross-section fibers such as Kodel (trademark) 431 polyester (available from Eastman Chemical Products, Inc., Kingsport, Tennessee, U.S.A.). The aforementioned disclosure attempts to solve the problem of gel blocking which occurs in some absorbant products where the superabsorbant polymer in granule form is layered within absorbant core. As the superabsorbant polymer granules absorb fluid they swell. Liquid transport through the swollen gel is limited primarily to the slow rates of diffusion. The European Patent Application 0 188 091 attempts to solve this barrier problem of swollen gels by uniformly dispersing the superabsorbant polymer throughout the web as a uniformly thin coated film on the fibers. The claim is that they will not block fluid transport throughout the remainder of the open network structure of the web. The thinly coated fibers of 0 188 091 only absorb fluid in the coating. They do not wick fluid. These webs are dependent on the capillary action of the pores between the fibers for wicking action. The coated fibers, filaments, or webs coated with superabsorbant polymer of this invention unexpectedly both wick and absorb fluid.

The problem of blocking capillary wicking action between superabsorbant coated hydrophilic fiber base materials discussed in U.S. Patent 4,721,647 is not a problem in the present invention since the wicking action does not depend solely on capillary action between filaments. Furthermore, substantial uniform coating of monomer solution is accomplished within the grooves of the base fibers as opposed to outer perimeter and between filaments of the prior art hydrophilic fibers.

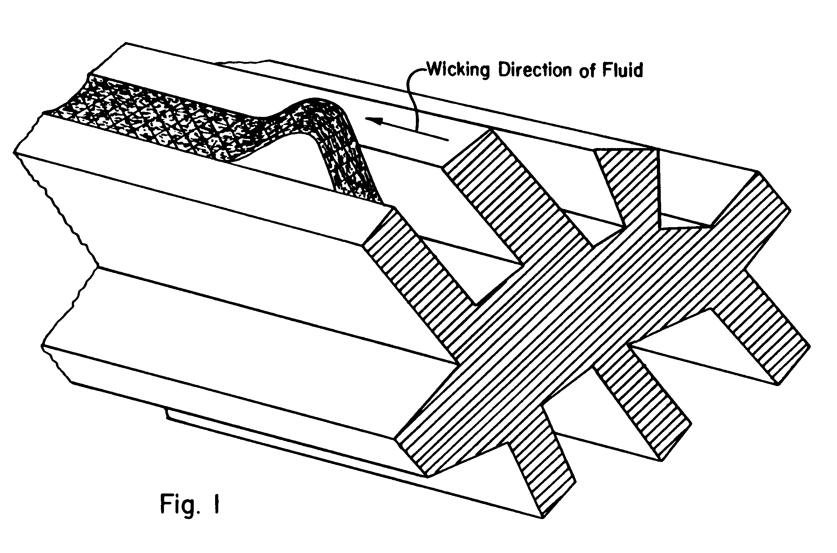

The liquid transport of the base fibers is attributed to having a desired combination of hydrophilic coating and surface geometry. One would expect that coating these fibers with superabsorbant polymer (especially using amounts of polymer which completely fill the grooves of the base fibers) would destroy the preferred geometry of the filament necessary for liquid transport. Unexpectedly when these base fibers which have been coated with super-absorbant polymer are subjected to a fluid such as water or synthetic urine, the superabsorbant polymer filling the grooves is observed to swell as it absorbs fluid. The swollen gel pops out of the grooves sufficient to allow the fluid to wick down the open groove until it contacts additional superabsorbant. The process is repeated continuously until the super-absorbant is consumed or the end of the filament is reached. Although it is not desired to be bound by any particular mechanism, it is believed that the hydrophilic coating initially placed on the base fibers is not destroyed by the superabsorbant polymer coating and the desired geometry of the grooves is restored as the superabsorbant polymer swells and moves out of the grooves. Also it is believed that no bonding occurs between the superabsorbant polymer coating and the fiber surface to hold the gel. A cross-sectional schematic of a single base fiber having a groove filled with superabsorbant polymer is shown in Figure 1. The superabsorbant polymer is shown as it swells and pops out of the groove. This action allows room for more fluid to enter and wick in the groove.

The base fibers can be comprised of any material known in the art capable of having a cross-section of the desired geometry. Preferred materials for use in the present invention are polyesters.

The preferred polyester materials useful in the present invention are polyesters or copolyesters that are well known in the art and can be prepared using standard techniques, such as, by polymerizing dicarboxylic acids or esters thereof and glycols. The dicarboxylic acid compounds used in the production of polyesters and copolyesters are well known to those skilled in the art and illustratively include terephthalic acid, isophthalic acid, p,p'-diphenyldicarboxylic acid, p,p'-dicarboxydiphenyl ethane, p,p'-dicarboxydiphenyl hexane, p,p'-dicarboxydiphenyl ether, p,p'-dicarboxyphenoxy ethane, and the like, and the dialkylesters thereof that contain from 1 to about 5 carbon atoms in the alkyl groups thereof.

Suitable aliphatic glycols for the production of polyesters and copolyesters are the acyclic and alicyclic aliphatic glycols having from 2 to 10 carbon atoms, especially those represented by the general formula $HO(CH_2)_pOH$, wherein p is an integer having a value of from 2 to about 10, such as ethylene glycol, trimethylene glycol, tetramethylene glycol, and pentamethylene glycol, decamethylene glycol, and the like.

Other known suitable aliphatic glycols include 1,4-cyclohexanedimethanol, 3-ethyl-1,5-pentanediol, 1,4-xylylene, glycol, 2,2,4,4-tetramethyl-1,3-cyclobutanediol, and the like. One can also have present a hydroxylcarboxyl compound such as 4,-hydroxybenzoic acid, 4-hydroxyethoxybenzoic acid, or any of the other hydroxylcarboxyl compounds known as useful to those skilled in the art.

It is also known that mixtures of the above dicarboxylic acid compounds or mixtures of the aliphatic glycols can be used and that a minor amount of the dicarboxylic acid component, generally up to about 10 mole percent, can be replaced by other acids or modifiers such as adipic acid, sebacic acid, or the esters thereof, or with modifiers that impart improved dyeability to the polymers. In addition one can also include pigments, delusterants or optical brighteners by the known procedures and in the known amounts.

The most preferred polyester for use in preparing the base fiber is poly(ethylene terephthalate) (PET).

Other materials that can be used to make the base fibers include polyamides such as a nylon, e.g., nylon 66 or nylon 6; polypropylene; polyethylene; and cellulose esters such as cellulose triacetate or cellulose diacetate.

A single base fiber or coated fiber of the present invention preferably has a denier of between about 3 and about 1,000 (about $3.33 \times 10^{-7}$ and about $1.11 \times 10^{-4}$ kg/m), more preferred is between about 10 and about 70 (about $1.11 \times 10^{-6}$ and about $7.78 \times 10^{-6}$ kg/m).

The base fibers preferably have a surface treatment applied thereto (prior to coating with the super-absorbant polymer). Such surface treatment may or may not be critical to obtain the required spontaneous transportability property. The nature and criticality of such surface treatment for any given fiber can be determined by a skilled artisan through routine experimentation using techniques known in the art and/or disclosed herein. A preferred surface treatment is a coating of a hydrophilic lubricant on the surface of the fiber. Such coating is typically uniformly applied at about a level of at least 0.05 weight percent, with about 0.1 to about 2 weight percent being preferred. Preferred hydrophilic lubricants include a potassium lauryl phosphate based lubricant comprising about 70 weight percent poly(ethylene glycol) 600 monolaurate. Another surface treatment is to subject the fibers to oxygen plasma treatment, as taught in, for example, Plastics Finishing and Decoration, Chapter 4, Ed. Don Satas, Van Nostrand Reinhold Company (1986).

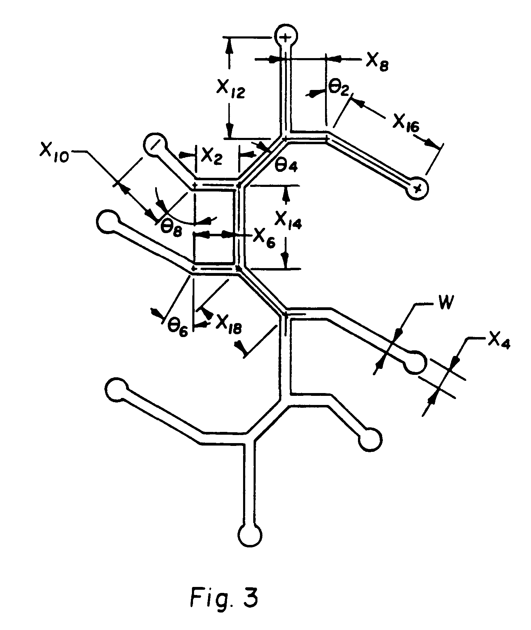

Figures 3 through 8 illustrate spinneret orifices which will prepare fibers of a geometry suitable for use in the present invention.

In Figure 3, W is between 0.064 millimeters (mm) and 0.12 mm. $X_2$ is $4W\ ^{+4W}_{-1W}$; $X_4$ is $2W \pm 0.5W$; $X_6$ is $6W\ ^{+4W}_{-2W}$; $X_8$ is $6W\ ^{+5W}_{-2W}$; $X_{10}$ is $7W\ ^{+5W}_{-2W}$; $X_{12}$ is $9W\ ^{+5W}_{-1W}$; $X_{14}$ is $10W\ ^{+5W}_{-2W}$; $X_{16}$ is $11W\ ^{+5W}_{-2W}$; $X_{18}$ is $6W\ ^{+5W}_{-2W}$; $\theta_2$ is $30° \pm 30°$; $\theta_4$ is $45° \pm 45°$; $\theta_6$ is $30° \pm 30°$; and $\theta_8$ is $45° \pm 45°$.

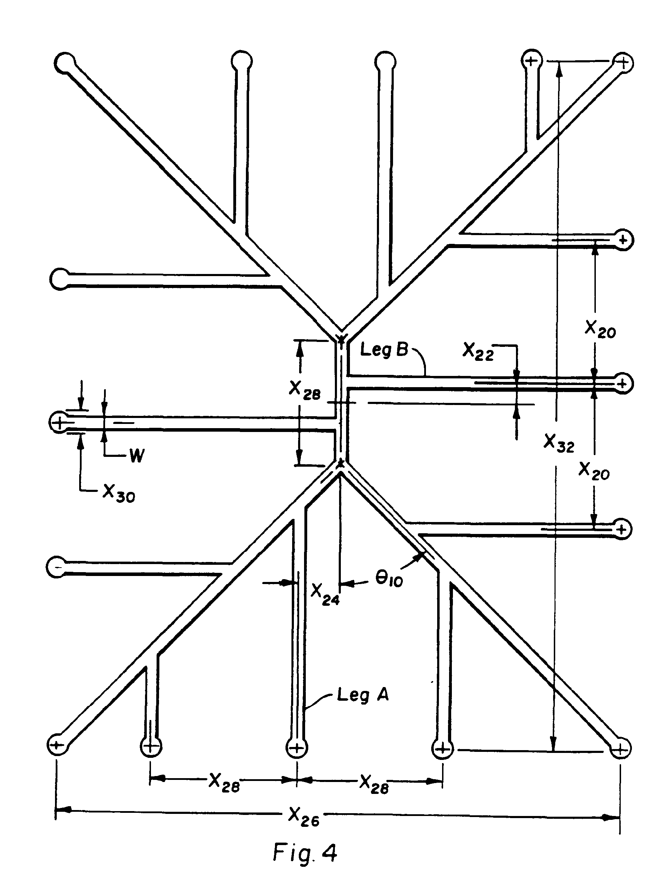

In Figure 4, W is between 0.064 mm and 0.12 mm; $X_{20}$ is $17W\ ^{+5W}_{-2W}$; $X_{22}$ is $3W \pm W$; $X_{24}$ is $4W \pm 2W$; $X_{26}$ is $60W\ ^{+8W}_{-4W}$; $X_{28}$ is $17W\ ^{+5W}_{-2W}$; $X_{30}$ is $2W \pm 0.5W$; $X_{32}$ is $72W\ ^{+10W}_{-5W}$; and $\theta_{10}$ is $45° \pm 15°$. In addition, each Leg B can vary in length from 0 to $\dfrac{X_{26}}{2}$; and each Leg A can vary in length from 0 to

$$\tan\ (90-\theta_{10}) \left[ \frac{X_{26}}{2} - X_{24} \right] .$$

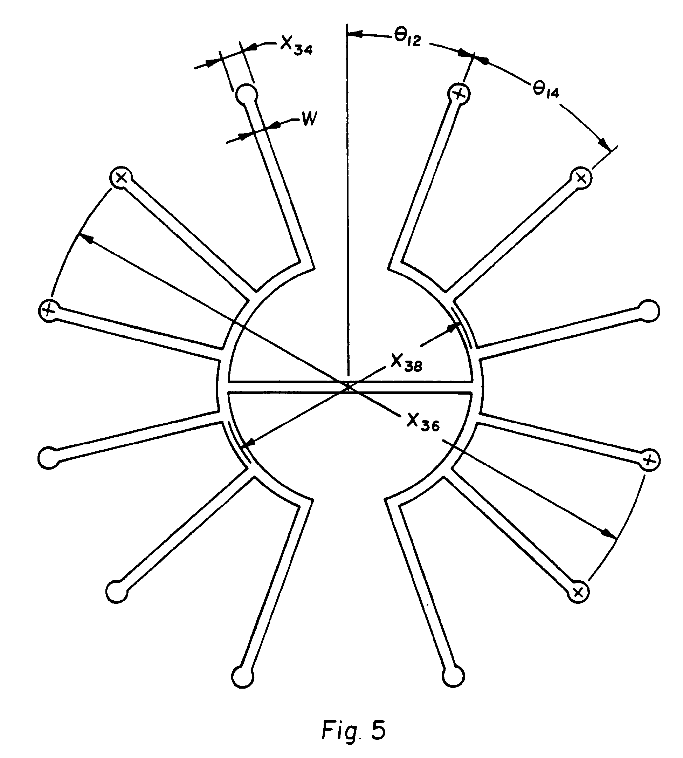

In Figure 5, W is between 0.064 mm and 0.12 mm; $X_{34}$ is $2W \pm 0.5W$; $X_{36}$ is $58W\ ^{-20W}_{-10W}$; $X_{38}$ is $24W\ ^{+20W}_{-6W}$; $\theta_{12}$ is $20°\ ^{+15W}_{-10W}$; $\theta_{14}$ is $\dfrac{180° - 2\theta_{12}}{n - 1}$; and n = number of legs per 180° = 2 to 6.

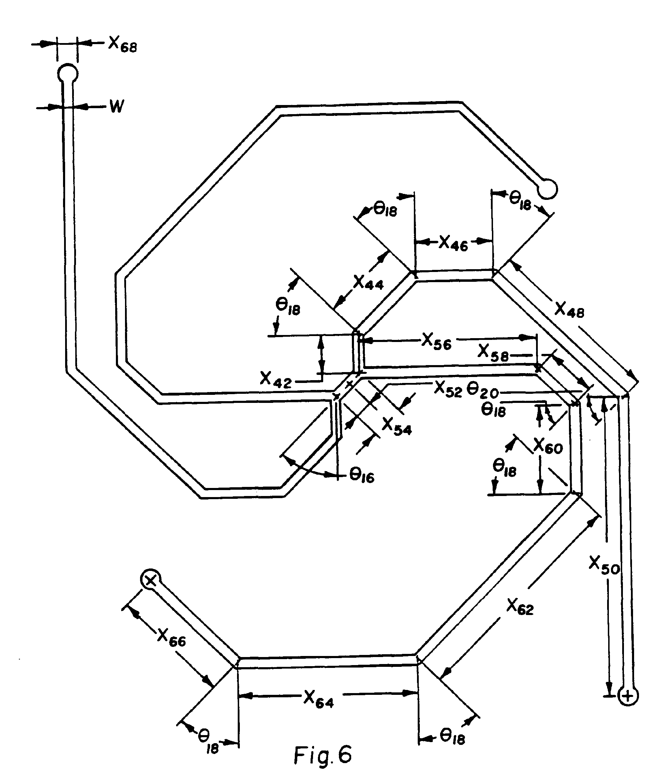

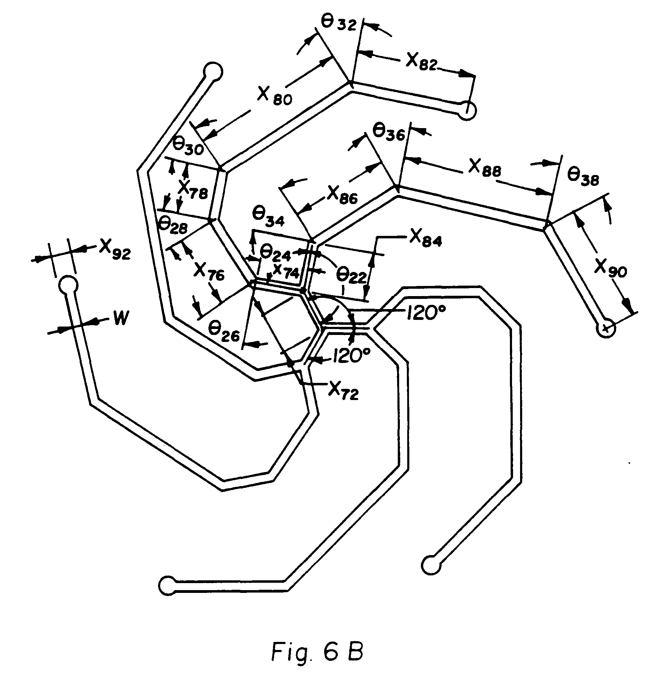

In Figure 6, W is between 0.064 mm and 0.12 mm; $X_{42}$ is $6W\ ^{+4W}_{-2W}$; $X_{44}$ is $11W \pm 5W$; $X_{46}$ is $11W \pm 5W$; $X_{48}$ is $24W \pm 10W$; $X_{50}$ is $38W \pm 13W$; $X_{82}$ is $3W\ ^{+3W}_{-1W}$; $X_{54}$ is $6W\ ^{+6W}_{-1W}$; $X_{86}$ is $11W \pm 5W$; $X_{88}$ is $7W \pm 5W$; $X_{80}$ is $17W \pm 7W$; $X_{82}$ is $28W \pm 11W$; $X_{64}$ is $24W \pm 10W$; $X_{66}$ is $17W \pm 7W$; $X_{88}$ is $2W \pm 0.5W$; $\theta_{16}$ is $45°\ ^{+30°}_{-15°}$; $\theta_{18}$ is $45° \pm 15°$; and $\theta_{20}$ is $45° \pm 15°$.

In Figure 6B W is between 0.064 mm and 0.12 mm, $X_{72}$ is $8W\ ^{+4W}_{-2W}$, $X_{74}$ is $8W\ ^{+4W}_{-2W}$, $X_{76}$ is $12W \pm 4W$, $X_{78}$ is $8W \pm 4W$, $X_{80}$ is $24W \pm 12W$, $X_{82}$ is $18W \pm 6W$, $X_{84}$ is $8W\ ^{+4W}_{-2W}$ $X_{86}$ is $16W \pm 6W$, $X_{88}$ is $24W \pm 12W$, $X_{90}$ is $18W \pm 6W$, $X_{92}$ is $2W \pm 0.5w$, $\theta_{22}$ is $135° \pm 30°$, $\theta_{24}$ is $90° \pm\ ^{45°}_{30°}$, $\theta_{26}$ is $45° \pm 15°$, $\theta_{28}$ is $45° \pm 15°$, $\theta_{30}$ is $45° \pm 15°$, $\theta_{32}$ is $45° \pm 15°$, $\theta_{84}$ is $45° \pm 15°$, $\theta_{36}$ is $45° \pm 15°$, and $\theta_{38}$ is $45° \pm 15°$.

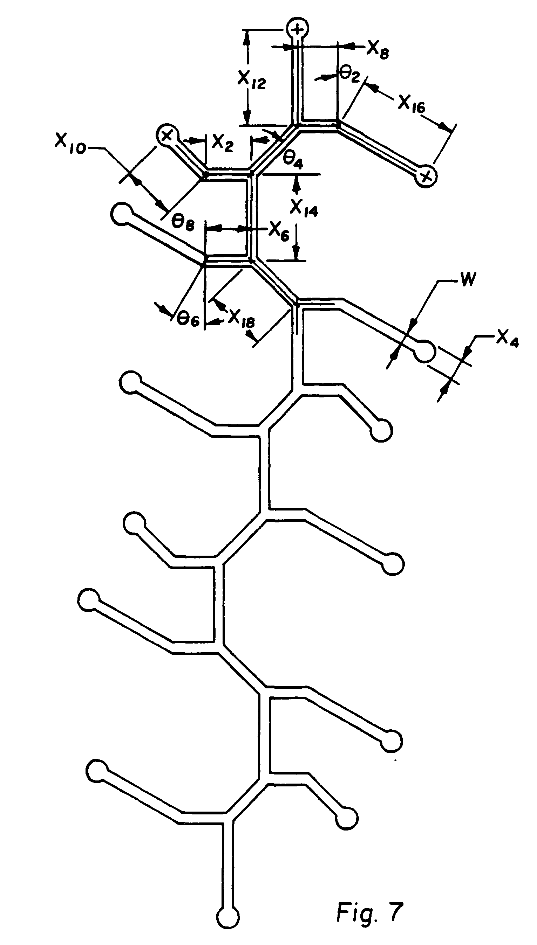

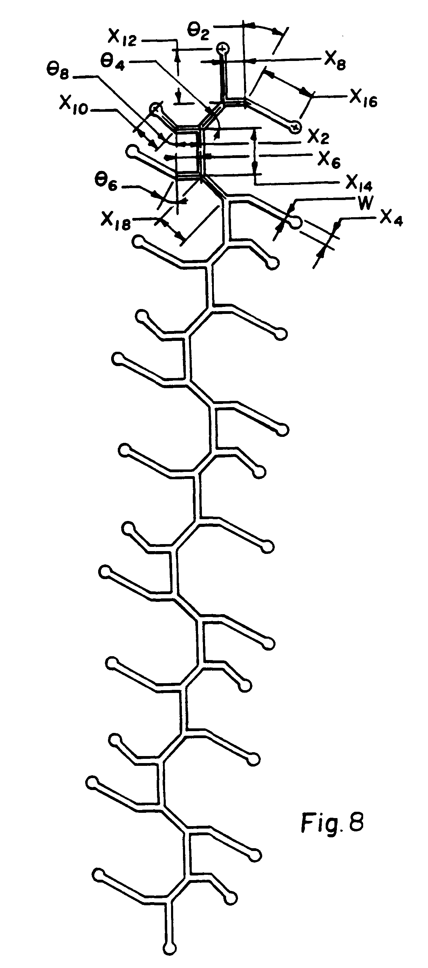

In Figure 7, the depicted spinneret orifice contains two repeat units of the spinneret orifice depicted in Figure 3, therefore, the same dimensions for Figure 3 apply to Figure 7. Likewise, in Figure 8, the depicted spinneret orifice contains four repeat units of the spinneret orifice depicted in Figure 3, therefore, the same dimen-

EP 0 536 308 B1

sion for Figure 3 applies to Figure 8.

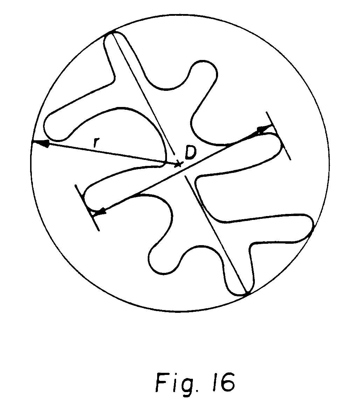

Figure 16 illustrates the method for determining the shape factor, X, of the fiber cross-section. In Figure 16, r = 37.5 mm, $P_w$ = 355.1 mm, D = 49.6 mm; thus, for the fiber cross-section of Figure 16:

$$X = \frac{355.1}{4 \times 37.5 + (\pi - 2)\,49.6} = 1.72\,.$$

The coated fibers of the present invention are preferably incorporated into an absorbant article in which it is desired to move or transport aqueous fluids. Such absorbant articles include, but are not limited to, diapers, incontinence pads, feminine hygiene articles such as tampons, wipes, and the like. By use of the coated fibers of the invention, the prior art problem of leaking can be eliminated or at least minimized.

The coated fibers of the present invention can be in the form of crimped or uncrimped tows, webs, or staple fibers comprising a plurality of the coated fibers of the present invention. A tow of the invention preferably has a denier of about 10,000 to about 400,000 ($1.11 \times 10^{-3}$ to $4.44 \times 10^{-2}$ kg/m).

An absorbant article of the present invention comprises two or more coated fibers of the present invention wherein at least part of said fibers are located near the center of said absorbant article and at least part of same said fibers are located away from the center of said absorbant article; and wherein said fibers are capable of being in contact with an aqueous fluid for about at least 10 seconds near the center of said absorbant article. As used in this context, "near the center" of the absorbant article means the geometric center and the area consisting of 50 area % of the total article immediately surrounding said geometric center; "away from the center" of the absorbant article means the remaining 50 area % that is not near the center of the article. In addition, other sinks (i.e., other than the superabsorbant polymer coating) optionally may be in contact with the coated fibers of the present invention. Preferred other sinks are fluff pulp, superabsorbant material, and combinations thereof. It is preferred that such other sinks are in contact with a given fiber near the end of such fiber in the area away from the center of the article. As used in this context the term "near the end" of a fiber refers to an actual end of a fiber or the area consisting of the end 10% of the length of the fiber.

A preferred absorbant article of the present invention comprises a diaper or incontinent pad having a major axis and a minor axis and a length in excess of a width which comprises a top sheet, a back sheet, and an absorbant core comprising at least one absorbant layer wherein said article further comprises the tow of the present invention. The tow may be crimped or uncrimped.

The tow in said absorbant article can be located in several different positions with several different spatial orientations. For example, the tow can be uniformly spread across all or part of the width of the article and the fibers of the tow can be substantially parallel to the major axis of the article and extend from about 1/2 to substantially the length of the article.

Alternatively, the fibers of the tow can be substantially parallel to the major axis of the diaper and extend substantially the length of the diaper.

By use of a tow of the coated fibers of the invention in an absorbant article such as a diaper, urine can be transported to a larger surface area on the diaper. Thus, the amount of superabsorbant material required in the diaper can be reduced and the diaper surface will be drier.

By utilizing the fibers of the present invention in a diaper construction, it is preferred that at least one of the following benefits be realized.

(i) The effective surface area of the diaper utilized for urine/aqueous fluid movement will increase by 5% to 30%.

(ii) The amount of superabsorbant material utilized in the diaper will reduce by 2% to 25%.

(iii) The diaper will be thinner by about 2% to 15%.

(iv) The strikethrough (seconds)/rewet (grams) responses as measured by the strikethrough/rewet test described in U.S. Patent 4,324,247 are improved with the strikethrough being reduced from about 2 to about 50% and the rewet being reduced from about 2 to about 70% when compared to equivalent structures without the fibers (tow) of this invention being present. This results in the interface between the diaper and the wearer remaining drier.

The coated fibers of the tow can be located in the absorbant article at any place which will result in an overall beneficial effect. For example, the fibers can be located between the top sheet and the absorbant core, incorporated into the absorbant core, between the absorbant core and the back sheet, or multiple combinations of the above.

The top sheet of the absorbant article of the present invention can be made of any material known in the art for such use. Such materials include, but are not limited to, polypropylene, polyethylene, polyethylene terephthalate, cellulose or rayon; preferred is polypropylene. The top sheet is the sheet which is designed to be in contact with the body during typical end uses. Such a top sheet is alternatively referred to in the art as a "facing sheet," and is typically comprised of a web of short and/or long fibers.

The back sheet of the absorbant article of the present invention can be made of any material known in

8

EP 0 536 308 B1

the art for such use. Such materials include, but are not limited to, polyethylene, a polyester, or polypropylene; preferred is polyethylene. The back sheet is typically impervious to body fluids such as urine.

The absorbant core of the absorbant article of the present invention preferably comprises fluff pulp and, optionally, superabsorbant powder. Fluff pulp is used extensively in the art. Fluff pulp is a batt formed of loosely compacted short cellulose fibers, such as wood pulp fibers, or cotton linters, or mixtures thereof, which are primarily held together by interfiber bonds usually requiring no added adhesive although thermoplastic binder(s) may also be used. This batt is a low density coherent web of loosely compacted fibers preferably comminuted wood pulp fibers. Examples of absorbant powder are polyacrylates, acrylic acid based polymers, saponified starch, and polyacrylonitrile graft copolymers.

Other preferred embodiments of the absorbant article of the present invention include wherein the coated fibers of the tow are tightly compacted in the impingement zone such that the fibers are substantially in contact with each other, and toward each end of the length of the article the fibers of the tow flare and are substantially not in contact with each other. In addition, the tow can have from one half to ten turns of twist in the impingement zone. The terms "impingement zone", "impinging area", and like terms refer to that area or zone where body fluid first contacts or impinges the absorbant article during its intended use. The impingement zone may be near the center of the absorbant article, away from the center, or overlapping both areas.

It is also contemplated that the coated fibers of the present invention can be in the form of staple fiber which may or may not be crimped. When in the form of staple fiber, a preferred absorbant article of the present invention comprises a diaper or incontinent pad having a major axis and a minor axis and a length in excess of a width comprising a top sheet, a back sheet, and an absorbant core comprising at least one absorbant layer wherein said core comprises an intimate blend of the staple fiber of the present invention.

Another preferred embodiment of the absorbant article of the present invention is wherein the article contains up to three tows of the invention and wherein the major axis of each tow lies between ±30° around the major axis of the article and wherein the tows lie either just beneath the top sheet or lie intimately mixed with the absorbant core or lie adjacent to the back sheet.

Another preferred embodiment of the absorbant article of the present invention is a two piece diaper wherein one piece contains tow of the invention and receives the impinging fluid during the diaper's intended use and is reusable, and wherein the second piece is a fluid storage element and is replaceable.

The absorbant article of this invention can optionally contain a tissue or low density spacer layer which is adjacent to the top sheet between the top sheet and absorbant core. In such case the tow preferably lies between the absorbant core and said tissue or density spacer.

In still another preferred embodiment of the absorbant article of the present invention the coated fibers of the tow are in intimate contact with part of the absorbant core located away from the impingement zone.

Other absorbant articles contemplated by the present invention (which may or may not have a specific impingement zone) in which the coated fibers of the present invention can be beneficial include, but are not limited to, a surgical sponge, a wound dressing, a sweat absorbing insole for footwear, and the like.

The base fibers of the present invention can be prepared by techniques taught in Serial No. 333,651 and/or as taught herein.

The absorbant articles of the present invention can be made by use of techniques known in the art, for example in U.S. Patents 4,573,986; 3,938,522; 4,102,340; 4,044,768; 4,282,874; 4,285,342; 4,333,463; 4,731,066; 4,681,577; 4,685,914; and 4,654,040; and/or by techniques disclosed herein. The tow of the present invention can be incorporated into the absorbant article at any location which will improve fluid movement so as to better utilize the absorbant materials of the article.

The following examples are to illustrate the invention but should not be interpreted as a limitation thereon.

EXAMPLES

Example 1 (Base Fiber Preparation)

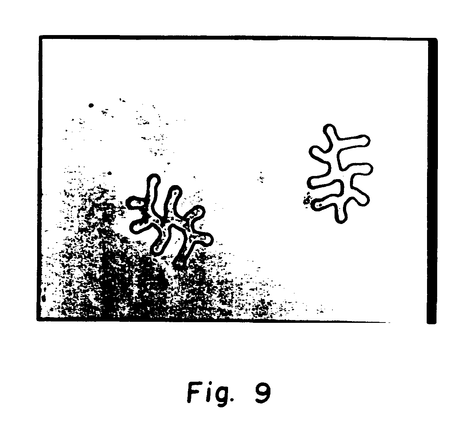

Poly(ethylene terephthalate) (PET) polymer of 0.6 I.V. was used in this example. I.V. is the inherent viscosity as measured at 25°C at a polymer concentration of 0.50 g/100 milliliters (mL) in a suitable solvent such as a mixture of 60% phenol and 40% tetrachloroethane by weight. The polymer was dried to a moisture level of $\leqq$0.003 weight percent in a Patterson Conaform dryer at 120°C for a period of 8 hours. The polymer was extruded at 283°C through an Egan extruder, 1.5-inch (38.16 mm) diameter, with a length to diameter ratio of 28:1. The fiber was extruded through an eight orifice spinneret wherein each orifice is as shown in Figure 3 wherein W is 0.084 mm, $X_2$ is 4W, $X_4$ is 2W, $X_6$ is 6W, $X_8$ is 6W, $X_{10}$ is 7W, $X_{12}$ is 9W, $X_{14}$ is 10W, $X_{16}$ is 11W, $X_{18}$ is 6W, $\theta_2$ is 0°, $\theta_4$ is 45°, $\theta_6$ is 30°, and $\theta_8$ is 45°. The polymer throughput was about 7 pounds (lb)/hour (3.18 kg/hour). The air quench system has a cross-flow configuration. The quench air velocity at the top of the

9

screen was an average of 294 feet (ft)/minute (89.61 m/minute). At a distance of about 7 inches (177.8 mm) from the top of the screen the average velocity of the quench air was about 285 ft/minute (86.87 m/minute), and at a distance of about 14 inches (355.6 mm) from the top of the screen the average quench air velocity was about 279 ft/minute (85.04 m/minute). At about 21 inches (533.4 mm) from the top of the air screen the average air velocity was about 340 ft/minute (103.63 m/minute). The rest of the screen was blocked. Spinning lubricant was applied via ceramic kiss rolls. The lubricant has a general composition as follows: it is a potassium lauryl phosphate (PLP) based lubricant having poly(ethylene glycol) 600 monolaurate (70% by weight) and polyoxyethylene (5) potassium lauryl phosphate (30% by weight). An emulsion of the above lubricant with water (90%) was used as the spinning lubricant. The lubricant level on the fiber samples was about 1.5%. Fibers of 20 dpf (denier per filament) (22.22 dtex) were wound at 3,000 meters per minute (MPM) on a Barmag SW4SL winder. A photomicrograph of a cross-section of this fiber is shown in Figure 9 (150x magnification). The single fiber was tested for spontaneous surface transportation of an aqueous solution which was aqueous Syltint Poly Red (obtained from Milliken Chemicals) which is 80 weight % water and 20 weight % red colorant. The single fiber of 20 dpf (22.22 dtex) spontaneously surface transported the above aqueous solution. The following denier per filament (dtex) PET fibers were also made at different speeds as shown in Table 1 below:

## Table 1

| dpf (dtex) | Spin Speed (MPM) | Winder |
|---|---|---|
| 20 (22.22) | 3,000 | Barmag |
| 40 (44.44) | 1,500 | Leesona |
| 60 (66.67) | 1,000 | Leesona |
| 120 (133.33) | 500 | Leesona |
| 240 (266.67) | 225 | Leesona |
| 400 (444.44) | 150 | Leesona |

All the single fibers of above PET fiber with the dpf of 20, 40, 60, 120, 240, and 400 spontaneously surface transported the aqueous solution of Syltint Poly Red liquid. The value of the "X" parameter (as defined hereinbefore) for these fibers was about 1.7. PET film of 0.02 inch (0.508 mm) thickness was compression molded from the same polymer as that used for making the above fiber. Contact angle of distilled water on the above film was measured in air with a contact angle goniometer. The contact angle was 71.7°. Another sample of the same film as above was sprayed with the same lubricant as used for making the fiber in this example at about 1.5% level. The contact angle of distilled water on the PET film sprayed with the lubricant was about 7°. Thus, the factor $(1 - X \cos \theta)$ in this case is $(1 - 1.7(\cos 7°)) = -0.69$, which is less than zero.

Example 2 (Base Fiber Preparation)

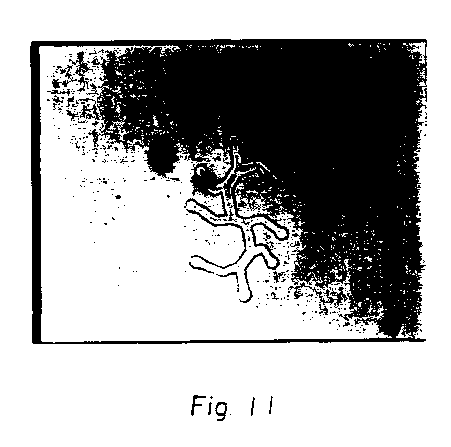

Polyhexamethylene adipamide (nylon 66) was obtained from Du Pont [Zytel (trademark) 42]. The polymer was extruded at 279°C. A spinneret as shown in Figure 3 was used to form 46 dpf (51.11 dtex) fiber at 255 meters/minute speed. The specific dimensions of the spinneret orifices were the same as described in Example 1 except that $\theta_2$ was 30° instead of 0°. The quenching conditions were the same as those for obtaining PET fiber as in Example 1. A photomicrograph of the fiber cross-section is shown in Figure 11 (150x magnification). The lubricant level on the fiber was about 1.8% by weight. The same lubricant as used in the PET fiber was used (Example 1). This nylon 66 fiber spontaneously transported the aqueous Syltint Poly Red solution on the fiber surface. The value of the "X" parameter for this fiber was about 1.9. Nylon 66 film of 0.02 inch (0.508 mm) thickness was compression molded from the same polymer as that used for making the fiber of Example 2. Contact angle of distilled water on the above film was measured in air with a contact angle goniometer. The contact angle was 64°. Another sample of the same film as above was sprayed with the same lubricant as used for making the fiber in this example at about the 1.8% level. The contact angle of distilled water on the nylon 66 film sprayed with the lubricant was about 2°. Thus, the factor $(1 - X \cos \theta)$ in this case is $(1 - 1.9(\cos 2°)) = -0.9$, which is less than zero.

Example 3 (Base Fiber Preparation)

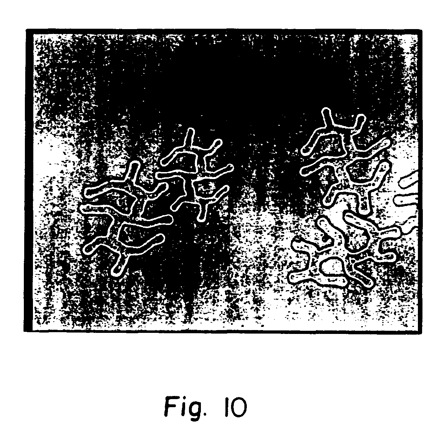

Polypropylene polymer was obtained from Shell Company (Grade 5C14). It was extruded at 279°C. A spinneret as shown in Figure 3 was used to form 51 dpf (56.67 dtex) fiber at 2,000 MPM speed. The specific dimensions of the spinneret orifices were the same as in Example 2. The quenching conditions were the same as those for obtaining PET fiber. A photomicrograph of the fiber cross-section is shown in Figure 10 (375x magnification). The lubricant level on the fiber was 2.6%. The same lubricant as used in PET fiber was used (Example 1). The polypropylene fiber spontaneously transported the aqueous Syltint Poly Red solution on the fiber surface. This spontaneously transportable phenomenon along the fiber surface was also observed for a 10 dpf (11.11 dtex), single polypropylene fiber. The value of the "X" parameter for this fiber was about 2.2. Polypropylene film of 0.02 inch (0.508 mm) thickness was compression molded from the same polymer as that used for making the above fiber of Example 3. Contact angle of distilled water on the above film was measured in air with a contact angle goniometer. The contact angle was about 110°. Another sample of the same film as above was sprayed with the same lubricant as used for making the fiber in this example at about the 2.6% level. The contact angle of distilled water on the polypropylene film sprayed with the lubricant was 12°. Thus, the factor (1-X cos θ) in this case is -1.1, which is less than zero.

Example 4 (Base Fiber Preparation)

Cellulose acetate (Eastman Grade CA 398-30, Class I) was blended with PEG 400 polymer and small quantities of antioxidant and thermal stabilizer. The blend was melt extruded at 270°C. A spinneret as shown in Figure 3 was used to form 115 dpf (127.78 dtex) fiber at 540 meters/minute speed. The specific dimensions of the spinneret orifices were the same as in Example 2. No forced quench air was used. The lubricant level on the fiber was 1.6%. The same lubricant as used in the PET fibers (Example 1) was used. The cellulose acetate fiber spontaneously transported the aqueous Syltint Poly Red solution on the fiber surface. The value of the "X" parameter for this fiber was about 1.8.

Example 5 (Comparative)

PET fiber of Example 1 was made without any spinning lubricant at 20 dpf (22.22 dtex). A single fiber did not spontaneously transport the aqueous Syltint Poly Red solution along the fiber surface.

Example 6 (Comparative)

PET fiber of circular cross-section was made. The denier per filament of the fiber was 20 (22.22 dtex). It had about 1.5% of the lubricant used in Example 1. A single fiber did not spontaneously transport the aqueous Syltint Poly Red solution along the fiber surface.

Example 7 (Base Fiber Preparation)

Poly(ethylene terephthalate) (PET) fiber of Example 5 (without any spinning lubricant) was treated with oxygen plasma for 30 seconds. Model "Plasmod" oxygen plasma equipment was used. Exciter power is provided by the RF generator operating at 13.56 MHz frequency. The plasma treatment was conducted at a constant level of 50 watts power. The oxygen plasma treated fiber spontaneously transported the aqueous Syltint Poly Red solution along the fiber. This fiber was tested again after washing five times and after 3 days and the spontaneously transportable behavior with the above aqueous solution was still observed. In order to determine the reduction in contact angle after the plasma treatment, a PET film of the same material as that of the fiber was subjected to the oxygen plasma treatment under the same conditions as those used for the fiber sample. The average contact angle of the oxygen plasma treated film with distilled water in air was observed to be 26° as measured by a contact angle goniometer. The corresponding contact angle for the control PET film (not exposed to the oxygen plasma) was 70°. The significant reduction in contact angle upon subjecting the untreated PET fiber to the oxygen plasma treatment renders it to be spontaneously surface transportable for aqueous solutions.

Example 8 (Base Fiber Preparation)

Poly(ethylene terephthalate) (PET) polymer of 0.6 IV was used in this example. It was extruded through a spinneret having eight orifices as shown in Figure 4 wherein W is 0.084 mm, $X_{20}$ is 17W, $X_{22}$ is 3W, $X_{24}$ is

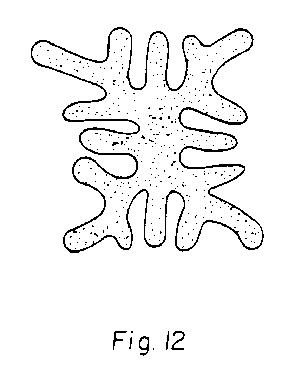

4W, $X_{26}$ is 60W, $X_{28}$ is 17W, $X_{30}$ is 2W, $X_{32}$ is 72W, $\theta_{10}$ is 45°, Leg B is 30W, and Leg A is 26W. The rest of the processing conditions were the same as those described in Example 1. A 100 dpf (111.11 dtex) fiber was spun at 600 MPM. A sketch of the cross-section of the fiber is shown in Figure 12. The lubricant level on the fiber was about 1%. The same lubricant as used in Example 1 was used. The above fiber spontaneously transported the aqueous Syltint Poly Red solution along the fiber surface. The value of the "X" parameter for this fiber was 1.5.

Example 9 (Base Fiber Preparation)

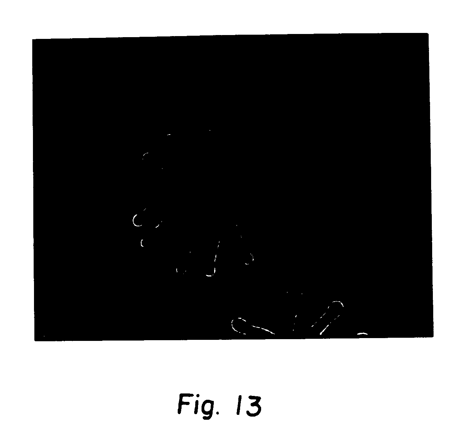

Poly(ethylene terephthalate) polymer of 0.6 IV was used in this example. It was extruded through a spinneret having eight orifices as shown in Figure 5 wherein W is 0.10 mm, $X_{34}$ is 2W, $X_{36}$ is 58W, $X_{38}$ is 24W, $\theta_{12}$ is 20°, $\theta_{14}$ is 28°, and n is 6. The rest of the extruding and spinning conditions were the same as those described in Example 1. A photomicrograph of the fiber cross-section is shown in Figure 13 (585x magnification). A 20 dpf (22.22 dtex) fiber was spun at 3000 MPM. The lubricant level on the fiber was about 1.7%. The same lubricant as used in Example 1 was used. The above fiber spontaneously transported the aqueous Syltint Poly Red solution along the fiber surface. The value of the "X" parameter for this fiber was about 2.4.

Example 10 (Base Fiber Preparation)

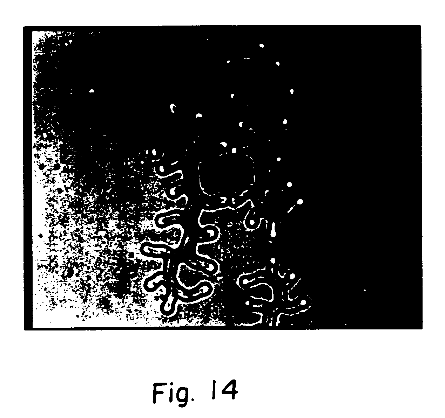

Poly(ethylene terephthalate) (PET) polymer of about 0.6 IV was used in this example. The polymer was extruded through a spinneret having four orifices as shown in Figure 7 wherein the dimensions of the orifices are repeats of the dimensions described in Example 2. The rest of the processing conditions were the same as those described in Example 1 unless otherwise stated. A 200 dpf (222.22 dtex) fiber was spun at 600 MPM. The polymer throughput was about 7 lbs/hr (3.18 kg/hr). An optical photomicrograph of the fiber is shown in Figure 14 (150x magnification). The lubricant level on the fiber was 2.0%. The same lubricant as used in Example 1 was used. The above fiber spontaneously transported the aqueous Syltint Poly Red solution along the fiber surface. The value of the "X" parameter for this fiber was about 2.2.

Example 11 (Base Fiber Preparation)

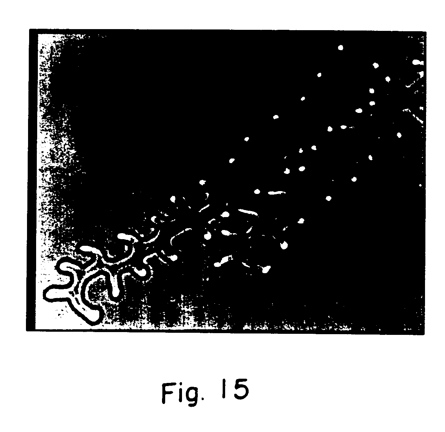

Poly(ethylene terephthalate) (PET) polymer of 0.6 IV was used in this example. The polymer was extruded through a spinneret having two orifices as shown in Figure 8 wherein the dimensions of the orifices are repeats of the dimensions described in Example 2. The rest of the processing conditions were the same as those described in Example 1. A 364 dpf (404.44 dtex) fiber was spun at 600 MPM. The cross-section of the fiber is shown in Figure 15 (150x magnification). The lubricant level on the fiber was about 2.7%. The same lubricant as used in Example 1 was used. The above fiber spontaneously transported the aqueous Syltint Poly Red solution along the fiber surface. The value of the "X" parameter for this fiber was 2.1.

Example 12 (Base Fiber Preparation)

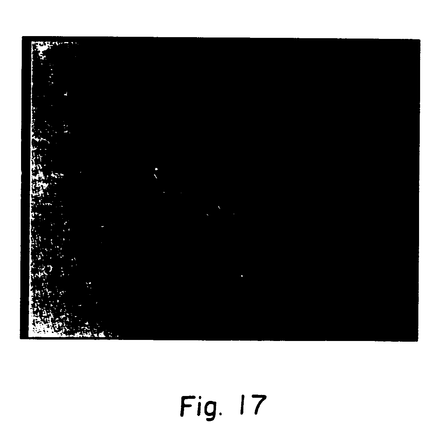

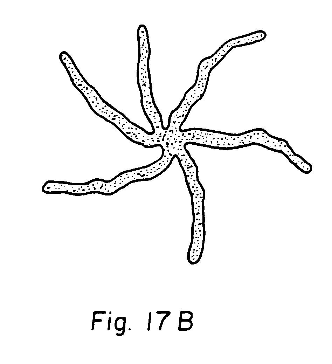

Poly(ethylene terephthalate) (PET) polymer of 0.6 IV was used in this example. It was extruded through a spinneret having eight orifices as shown in Figure 6 wherein W is 0.10 mm, $X_{42}$ is 6W, $X_{44}$ is 11W, $X_{46}$ is 11W, $X_{48}$ is 24W, $X_{50}$ is 38W, $X_{52}$ is 3W, $X_{54}$ is 6W, $X_{56}$ is 11W, $X_{58}$ is 7W, $X_{60}$ is 17W, $X_{62}$ is 28W, $X_{64}$ is 24W, $X_{56}$ is 17W, $X_{58}$ is 2W, $\theta_{16}$ is 45°, $\theta_{18}$ is 45°, and $\theta_{20}$ is 45°. The rest of the processing conditions were the same as those described in Example 1. A 100 dpf (111.11 dtex) fiber was spun at 600 MPM. The cross-section of the fiber is shown in Figure 17. The lubricant level on the fiber was about 1%. The same lubricant as used in Example 1 was used. The above fiber spontaneously transported the aqueous Syltint Poly Red solution along the fiber surface. The value of the "X" parameter for this fiber was 1.3.

Example 13 (Base Fiber Preparation)

PET polymer of 0.6 I.V. is used in this example. It is extruded through a spinneret having 8 orifices as shown in Figure 6B wherein W is 0.10 mm, $X_{72}$ is 8W, $X_{74}$ is 8W, $X_{76}$ is 12W, $X_{78}$ is 8W, $X_{80}$ is 24W, $X_{52}$ is 18W, $X_{34}$ is 8W, $X_{56}$ is 16W, $X_{88}$ is 24W, $X_{90}$ is 18W, $X_{92}$ is 2W, $\theta_{22}$ is 135°, $\theta_{24}$ is 90°, $\theta_{26}$ is 45°, $\theta_{28}$ is 45°, $\theta_{30}$ is 45°, $\theta_{32}$ is 45°, $\theta_{34}$ is 45°, $\theta_{36}$ is 45° and $\theta_{38}$ is 45°. A 20 dpf (22.22 dtex) fiber is spun at 3,000 m/min. The rest of the processing conditions are the same as those used in Example 1. The lubricant level on the fiber is about 1%. The cross-section of the fiber is shown in Figure 17B. This fiber spontaneously transports the aqueous Syltint Poly Red solution along the fiber surface. The "X" value for this fiber is about 2.1.

Example 14 (Example of the Invention)

500 cc of 45% by weight monomer concentration sodium acrylate/acrylic acid (75% degree of neutralization) water solution were prepared by adding sodium hydroxide to deionized water with ice cooling. Subsequently acrylic acid was added with ice cooling. Crosslinking agent (0.862 g) N-N'-methylene bis acrylamide and initiator (2.29 g) sodium persulfate were added at room temperature. The above solution was placed in a rotating "kiss" roll applicator. Continuous filament polyester yarn as prepared in Example 1 was coated with the above monomer solution with the applicator operating at 2 rpm and the yarn speed at 250 fpm (76.20 m/minute) as it contacted the applicator. A small package of yarn was wound (3 min) and placed in a nitrogen purged oven at 70°C overnight to polymerize the monomers. The yarn was removed and the wt % add on of polymer was determined to be 27%. The above coated yarns were placed in contact with tinted water (diluted Milliken Syltint Poly Red solution) and observed to simultaneously wick and absorb the fluid. The swollen gel was observed to move from the groove of the filament under microscopic observation.

Example 15 (Example of the Invention)

39 cc of distilled water were placed in a flask. 13.2 grams of sodium hydroxide were added with ice cooling to the flask. 30 cc of acrylic acid were added to form a clear solution with ice cooling. 0.115 g of N,N'-methylene bisacrylamide crosslinking agent and 0.316 g of sodium persulfate initiator were added with heating to 47°C for 10 minutes. A few single filaments of polyester yarn (15 cm) from yarn as prepared in Example 1 were coated with the above monomer solution only on one end (about 2.5 cm) with the remaining 12.5 cm left uncoated. The single filaments were placed in a nitrogen purged oven at 70°C for 20 minutes to polymerize the monomers. The single filaments were removed from the oven and subjected to tinted water in the uncoated portion. The fluid was observed to wick in the uncoated area without absorbing fluid until it reached the superabsorbant coated area where the fluid was absorbed by the superabsorbant polymer. As additional fluid was added the polymer formed a swollen gel which swelled out of the filament grooves thus allowing more fluid to wick to additional super-absorbant polymer along the filament. Similar results were observed when a single filament having the length of an average diaper [i.e., 15 inches (381.0 mm)] was coated with the monomer solution on-both ends of the filament with the remaining center portion [about 8 inches (203.2 mm)] left uncoated. After polymerizing the monomer solution on the filaments the uncoated center portion was subjected to the tinted water. The fluid wicked to the superabsorbant coated ends of the filament where it was absorbed by the superabsorbant polymer in the grooves of the coated fiber.

The invention has been described in detail with particular reference to preferred embodiments thereof, but it will be understood that variations and modifications can be effected within scope of the invention. All of the U.S. patents cited herein are hereby incorporated herein by reference in their entirety.

**Claims**

1. A synthetic coated fiber characterised in that the fibre has at least one continuous groove which is capable of spontaneously transporting water on the surface thereof wherein said fiber satisfies the equation

$$(1 - X \cos \theta_a) < 0,$$

   wherein

   $\theta_a$ is the advancing contact angle of water measured on a flat film made from the same material as the fiber and having the same surface treatment, if any,

   X is a shape factor of the fiber cross-section that satisfies the following equation

$$X = \frac{P_w}{4r + (\pi - 2)D}$$

   wherein

   $P_w$ is the wetted perimeter of the fiber and r is the radius of the circumscribed circle circumscribing the fiber cross-section and D is the minor axis dimension across the fiber cross-section, and wherein

   said fiber has coated thereon at least one superabsorbant polymer.

2. The coated fiber of Claim 1 wherein $2\frac{r}{D}$ is greater than 1.

3. The coated fiber of Claim 1 wherein $2\dfrac{r}{D}$ between 1.5 and 5.

4. The coated fiber of Claim 1 which satisfies the equation

$$\gamma_{LA} \cdot \frac{12\pi \cdot 10^{-4}}{\sqrt{\rho}} \cdot \sqrt{dpf} \cdot (1 - X \cos \theta_a) \leqq -0.3,$$

wherein $\gamma_{LA}$ is the surface tension of water in air in dynes/cm, $\rho$ is the fiber density in grams/cc, and dpf is the denier of the single fiber.

5. The coated fiber of Claim 1 wherein X is greater than 1.2.

6. The coated fiber of Claim 1 wherein X is greater than 2.5.

7. The coated fiber of Claim 1 wherein X is greater than 4.

8. The coated fiber of Claim 1 having a single fiber denier of between 3 and 1,000 ($3.33 \times 10^{-7}$ and $1.11 \times 10^{-4}$ kg/m).

9. The coated fiber of Claim 1 having a single fiber denier of between 10 and 70 ($1.11 \times 10^{-6}$ and $7.78 \times 10^{-6}$ kg/m).

10. The coated fiber of Claim 3 having a single fiber denier of between 10 and 70 ($1.11 \times 10^{-6}$ and $7.78 \times 10^{-6}$ kg/m).

11. The coated fiber of Claim 1 comprised of a material selected from the group consisting of a polyester, polypropylene, polyethylene, a cellulose ester, and a nylon.

12. The coated fiber of Claim 11 having coated thereon a layer of a hydrophilic lubricant between the fiber surface and the superabsorbant polymer coating.

13. The coated fiber of Claim 11 wherein said polyester is poly(ethylene terephthalate) and said hydrophilic lubricant is a potassium lauryl phosphate based lubricant comprising 70 weight percent poly(ethylene glycol) 600 monolaurate which is uniformly applied at a level of at least 0.05% by weight of the total fiber.

14. The coated fiber of Claim 1 comprised of a polyester having coated thereon a layer of a hydrophilic lubricant.

15. The coated fiber of Claim 1 wherein the width of each groove in the fiber cross-section at any depth in the groove is equal to or less than the width of the groove at its mouth.

16. The coated fiber of Claim 1 wherein said super-absorbant polymer is prepared from one or more monomers selected from acrylic acid, methacrylic acid, vinylsulfonic acid, vinylphosphonic acid, and a salt thereof.

17. The coated fiber of Claim 1 wherein the entire fiber is substantially coated with the super-absorbant polymer.

18. The coated fiber of Claim 1 wherein the fiber is coated intermittently with the superabsorbant polymer.

19. The coated fiber of Claim 1 wherein the fiber is substantially the length of an absorbant article and is coated on the ends of the fiber, but not in the center portion of the fiber.

20. An absorbant article comprising two or more coated fibers of Claim 1.

21. An absorbant article comprising two or more coated fibers of Claim 19.

22. The absorbant article of Claim 20 which is a diaper, an incontinent pad, or a feminine hygiene article.

23. A tow comprising a plurality of the coated fibers of Claim 1.

24. The tow of Claim 23 having a denier of 10,000 to 400,000 ($1.11 \times 10^{-3}$ to $4.44 \times 10^{-2}$ kg/m).

**25.** An absorbant article comprising the tow of Claim 23.


**Patentansprüche**

1. Synthetischer, beschichteter Faden, dadurch gekennzeichnet, daß der Faden mindestens eine kontinuierliche (endlose) Aussparung aufweist, die spontan Wasser auf der Oberfläche des Fadens zu transportieren vermag, wobei der Faden der folgenden Gleichung genügt:

$$(1 - X \cos \theta_a) < 0,$$

worin bedeuten:

$\theta_a$ den fortschreitenden Kontaktwinkel von Wasser, gemessen auf einer flachen Folie aus dem gleichen Material wie dem Faden und der gleichen Oberflächenbehandlung, sofern eine solche erfolgt ist,
X den Formfaktor des Fadenquerschnittes, der der folgenden Gleichung genügt:

$$X = \frac{P_w}{4r + (\pi - 2)D}$$

worin bedeuten:

$P_w$ der benetzte Umfang des Fadens und
r der Radius des Profilumkreises, der den Fadenquerschnittsabschnitt begrenzt und D die kleine Achsendimension über den Fadenquerschnittsabschnitt, und
wobei
der Faden mit mindestens einem superabsorbierenden Polymer beschichtet ist.


2. Beschichteter Faden nach Anspruch 1, bei dem $2\frac{r}{D}$ größer als 1 ist.


3. Beschichteter Faden nach Anspruch 1, bei dem $2\frac{r}{D}$ zwischen 1,5 und 5 liegt.


4. Beschichteter Faden nach Anspruch 1, der der folgenden Gleichung genügt:

$$\gamma_{LA} \cdot \frac{12\pi \cdot 10^{-4}}{\sqrt{\rho}} \cdot \sqrt{dpf} \cdot (1 - X \cos \theta_a) \leqq - 0,3,$$

worin $\gamma_{LA}$ die Oberflächenspannung von Wasser in Luft in Dyn/cm ist, $\rho$ die Fadendichte in Gramm/cc darstellt und dpf der Denier-Wert des einzelnen Fadens ist.


5. Beschichteter Faden nach Anspruch 1, in dem X größer als 1,2 ist.

6. Beschichteter Faden nach Anspruch 1, in dem X größer als 2,5 ist.

7. Beschichteter Faden nach Anspruch 1, in dem X größer als 4 ist.

8. Beschichteter Faden nach Anspruch 1, mit einem einzelnen Faden-Denier-Wert zwischen 3 und 1000 ($3,33 \times 10^{-7}$ und $1,11 \times 10^{-4}$ kg/m).

9. Beschichteter Faden nach Anspruch 1, mit einem einzelnen Faden-Denier-Wert zwischen 10 und 70 ($1,11 \times 10^{-6}$ und $7,78 \times 10^{-6}$ kg/m).

10. Beschichteter Faden nach Anspruch 3, mit einem einzelnen Faden-Denier-Wert zwischen 10 und 70 ($1,11 \times 10^{-6}$ und $7,78 \times 10^{-6}$ kg/m).

11. Beschichteter Faden nach Anspruch 1, aus einem Material ausgewählt aus der Gruppe bestehend aus einem Polyester, Polypropylen, Polyethylen, einem Celluloseester und einem Nylon.

12. Beschichteter Faden nach Anspruch 11, mit einer hierauf aufgetragenen Schicht aus einem hydrophilen Gleitmittel (Schmiermittel) zwischen der Fadenoberfläche und der superabsorbierenden Polymerbeschichtung.

13. Beschichteter Faden nach Anspruch 11, bei dem der Polyester ein Poly(ethylenterephthalat) ist und das hydrophile Gleitmittel (Schmiermittel) ein solches auf Basis eines Kaliumlaurylphosphates ist mit 70 Gew.-% Poly(ethylenglykol) 600-monolaurat, das gleichförmig in einer Konzentration von mindestens 0,05 Gew.-%, bezogen auf den Gesamtfaden, angewandt worden ist.

**14.** Beschichteter Faden nach Anspruch 1, aus einem Polyester, auf dem sich eine Schicht aus einem hydrophilen Gleitmittel (Schmiermittel) befindet.

**15.** Beschichteter Faden nach Anspruch 1, bei dem die Weite jeder Aussparung im Fadenquerschnittsabschnitt in einer beliebigen Tiefe der Aussparung gleich ist oder kleiner als die Weite der Aussparung an ihrer Ausmündung.

**16.** Beschichteter Faden nach Anspruch 1, bei dem das superabsorbierende Polymer hergestellt worden ist aus einem oder mehreren Monomeren, ausgewählt aus Acrylsäure, Methacrylsäure, Vinylsulfonsäure, Vinylphosphonsäure und einem Salz hiervon.

**17.** Beschichteter Faden nach Anspruch 1, bei dem praktisch der gesamte Faden mit dem superabsorbierenden Polymeren beschichtet ist.

**18.** Beschichteter Faden nach Anspruch 1, bei dem der Faden intermittierend mit dem superabsorbierenden Polymeren beschichtet ist.

**19.** Beschichteter Faden nach Anspruch 1, bei dem der Faden praktisch die Länge eines absorbierenden Artikels oder Gegenstandes aufweist und an den Enden des Fadens beschichtet ist, jedoch nicht im zentralen Bereich des Fadens.

**20.** Absorbierender Gegenstand mit zwei oder mehreren beschichteten Fäden nach Anspruch 1.

**21.** Absorbierender Gegenstand mit zwei oder mehreren beschichteten Fäden nach Anspruch 19.

**22.** Absorbierender Gegenstand nach Anspruch 20, bei dem es sich handelt um ein Diaper, eine Windel, einen Unpäßlichkeits-Bausch oder einen Hygieneartikel für Frauen.

**23.** Kabel (Endlosband) aus einer Vielzahl von beschichteten Fäden gemäß Anspruch 1.

**24.** Kabel nach Anspruch 23, mit einem Denier-Wert von 10000 bis 400000 (1,11 x 10$^{-3}$ bis 4,44 x 10$^{-2}$ kg/m).

**25.** Absorbierender Gegenstand mit dem Kabel nach Anspruch 23.

**Revendications**

**1.** Fibre synthétique enduite caractérisée en ce que la fibre présente au moins une gorge continue capable de transporter spontanément l'eau sur la surface de la fibre, fibre satisfaisant à l'équation

$$(1 - X \cos \theta_a) < 0,$$

où

$\theta_a$ est l'angle d'avancée de l'eau mesuré sur un film plat fait du même matériau que la fibre et ayant le même traitement de surface, s'il en a un,

X est le facteur de forme de la section transversale de la fibre conforme à l'équation suivante

$$X = \frac{R_w}{4r + (\pi - 2)D}$$

où

$P_w$ est le périmètre de la fibre mouillée et r est le rayon du cercle circonscrit à la section transversale de la fibre et D est la dimension du plus petit axe pris sur la section transversale de la fibre, et la fibre étant enduite d'au moins un polymère superabsorbant.

**2.** Fibre enduite selon la revendication 1, où 2r/D est supérieur à 1.

**3.** Fibre enduite selon la revendication 1, où 2r/D est compris entre 1,5 et 5.

**4.** Fibre enduite selon la revendication 1, conforme à l'équation.

$$\gamma_{LA} \cdot \frac{12\pi \cdot 10^{-4}}{\sqrt{\rho}} \cdot \sqrt{dpf} \cdot (1 - X \cos \theta_a) \leqq - 0.3,$$

où $\gamma_{LA}$ est la tension superficielle de l'eau dans l'air en dynes/cm, $\rho$ est la densité de la fibre en g/cc et dpf est le denier d'une seule fibre.

5. Fibre enduite selon la revendication 1, où X est supérieur à 1,2.

6. Fibre enduite selon la revendication 1, où X est supérieur à 2,5.

7. Fibre enduite selon la revendication 1, où X est supérieur à 4.

8. Fibre enduite selon la revendication 1, telle que le denier d'une seule fibre est compris entre 3 et 1000 ($3,33 \times 10^{-7}$ et $1,11 \times 10^{-4}$ kg/m).

9. Fibre enduite selon la revendication 1, telle que le denier d'une seule fibre est compris entre 10 et 70 ($1,11 \times 10^{-6}$ et $7,78 \times 10^{-6}$ kg/m).

10. Fibre enduite selon la revendication 3, telle que le denier d'une seule fibre est compris entre 10 et 70 ($1,11 \times 10^{-6}$ et $7,78 \times 10^{-6}$ kg/m).

11. Fibre enduite selon la revendication 1, faite d'un matériau choisi parmi un polyester, un polypropylène, un polyéthylène, un ester de cellulose et un nylon.

12. Fibre enduite selon la revendication 11, recouverte d'un lubrifiant hydrophile entre la surface de la fibre et le revêtement de polymère superabsorbant.

13. Fibre enduite selon la revendication 11, où le polyester est du polytéréphtalate d'éthylène, le lubrifiant hydrophile est un lubrifiant à base de lauryl phosphate de potassium comprenant 70% de polyéthylène glycol 600 monolaurate uniformément appliqué à un titre d'au moins 0,05% du poids total de la fibre.

14. Fibre enduite selon la revendication 1, comprenant un polyester recouvert d'une couche de lubrifiant hydrophile.

15. Fibre enduite selon la revendication 1, dans laquelle la largeur de chaque gorge dans la section transversale de la fibre à n'importe quelle profondeur de la gorge est inférieure ou égale à la largeur de la gorge prise sur son bord externe.

16. Fibre enduite selon la revendication 1, dans laquelle le polymère superabsorbant est préparé à partir d'un ou plusieurs monomères choisis parmi l'acide acrylique, l'acide méthacrylique, l'acide vinylsulfonique, l'acide vinylphosphonique ou les sels de ces derniers.

17. Fibre enduite selon la revendication 1, pratiquement entièrement enduite avec le polymère superabsorbant.

18. Fibre enduite selon la revendication 1, enduite de façon intermittente avec le polymère superabsorbant.

19. Fibre enduite selon la revendication 1, ayant pratiquement la longueur de l'article absorbant et enduite à ses extrémités, mais pas dans sa partie centrale.

20. Article absorbant comprenant deux fibres ou plus selon la revendication 1.

21. Article absorbant comprenant deux fibres ou plus selon la revendication 19.

22. Article absorbant selon la revendication 20, qui est une couche pour enfants ou incontinents ou un article d'hygiène féminine.

23. Câble comprenant plusieurs fibres enduites selon la revendication 1.

24. Câble selon la revendication 23, ayant un denier de 10 000 à 400 000 ($1,11 \times 10^{-3}$ et $4,44 \times 10^{-2}$ kg/m.)

25. Article absorbant comprenant le câble de la revendication 23.

Fig. 1

Time
t=0

LFA

LFA

Fig. 2A

t=t₁  LFA

LFA

Fig. 2B

t=t₂  LFA

LFA

Fig. 2C

Fig. 3

Fig. 4

Fig. 5

Fig. 6

Fig. 6 B

Fig. 7

Fig. 8

Fig. 9

*Fig.* 10

Fig. 11

Fig. 12

Fig. 13

Fig. 14

Fig. 15

Fig. 16

Fig. 17

Fig. 17 B